# EUROPEAN PATENT APPLICATION

(11) **EP 4 803 136 A1**
(43) Date of publication of application: **09.09.2026**
(21) Application number: 26162109.8
(22) Date of filing: 03.03.2026
(51) Int. Cl.: A61N 5/10

(54) **RADIATION THERAPY MACHINE INCLUDING BRAKE FOR GANTRY**

(30) Priority: 06.03.2025 US 202519072605
(71) Applicant: Varian Medical Systems, Inc., Palo Alto, CA 94304 (US)
(72) Inventor: WAGGONER, Thomas, Palo Alto, 94304 (US); GAUDIO, Stephen, Palo Alto, 94304 (US)
(74) Representative: Mathisen & Macara LLP

(57) **Abstract**

A radiation therapy machine 100 comprises a gantry 110 rotatable about an axis 132; a rotor 205b coupled to the gantry; and a brake 410. The brake includes braking arms 545 and an electric actuator 515, the electric actuator configured to cause the braking arms to engage the rotor based on a voltage applied to the electric actuator.

## Description

### TECHNICAL FIELD

Example embodiments relate to radiation therapy (radiotherapy) systems with a brake for a gantry.

### BACKGROUND

Radiation therapy (also called radiotherapy) is a cancer treatment that employs high doses of ionizing radiation, such as X-rays or high-energy electrons, protons, or other heavy charged particles, to kill cancer cells. Generally, radiation therapy is a localized treatment for a specific target tissue, such as a cancerous tumor. Ideally, radiation therapy is performed on a planning target volume (i.e., the target tissue) that spares the surrounding normal tissue from receiving doses above specified tolerances, thereby minimizing risk of damage to healthy tissue. For example, to accurately supply a planned radiation dose, the spatial distribution of delivered radiation dose within the patient must closely match the spatial distribution of the planned radiation dose. So that the planned radiation dose is correctly supplied to the planning target volume during radiation therapy, the patient should be correctly positioned relative to the radiation source that provides the radiation therapy. In addition, precisely controlling the position of the radiation source relative to the patient is a significant factor in accurately targeting tissue in the patient. In light of the above, drive systems that enable precise and repeatable rotational positioning of a radiation source about a patient are commonly employed in radiation therapy systems.

### SUMMARY

According to various embodiments, a radiation therapy machine comprises a gantry rotatable about an axis; a rotor coupled to the gantry; and at least one brake, the at least one brake including, braking arms, and an electric actuator, the electric actuator configured to cause the braking arms to engage the rotor based on a voltage applied to the electric actuator.

According to one or more example embodiments, the electric actuator includes a plunger, and a solenoid defining a channel to receive the plunger, the solenoid configured to cause the plunger to move based on the voltage applied to the electric actuator.

According to one or more example embodiments, the electric actuator is configured to move a portion of the plunger into the channel when a first voltage is applied to the electric actuator and is configured to maintain the position of the portion plunger in the channel when a second voltage is applied to the electric actuator, the second voltage being less than the first voltage.

According to one or more example embodiments, the electric actuator further includes a discharging circuit configured to discharge the voltage applied to the electric actuator, the discharging circuit including a Zener diode.

According to one or more example embodiments, the at least one brake further includes a linkage coupled to the plunger and the braking arms, the linkage configured to translate an axial force from the plunger to move the braking arms.

According to one or more example embodiments, the linkage is configured to translate a first axial force in a first direction into a second axial force in a second direction, the first axial force and the second axial force occurring at separate locations.

According to one or more example embodiments, the linkage includes a clevis, and the at least one brake further includes a nut between the clevis and the plunger.

According to one or more example embodiments, the nut prevents motion of the clevis in a first direction relative to the plunger.

According to one or more example embodiments, the linkage includes a clevis, and the at least one brake further includes a shaft, the clevis and the plunger coupled to the shaft such that a distance between the clevis and the plunger is adjustable.

According to one or more example embodiments, the at least one brake further includes at least one spring between the braking arms, the at least one spring configured to provide a clamping force to the braking arms.

According to one or more example embodiments, the at least one brake further includes an override mechanism configured to cause the braking arms to disengage the rotor independent of the voltage applied to the electric actuator.

According to one or more example embodiments, the rotor is between the braking arms.

According to one or more example embodiments, the electric actuator causes the braking arms to engage the rotor when the voltage applied to the electric actuator is zero volts.

According to one or more example embodiments, the radiation therapy machine further comprises a stand; and a drive system, the drive system coupling the stand to the gantry, the at least one brake being mounted to the drive system.

According to one or more example embodiments, the drive system includes a bearing assembly coupled to the gantry and the stand, the at least one brake being mounted to a portion of the bearing assembly.

According to one or more example embodiments, the at least one brake includes a plurality of brakes arranged around the rotor.

According to one or more example embodiments, the radiation therapy machine further comprises processing circuitry configured to cause the radiation therapy machine to, obtain a speed of the gantry, estimate a stopping distance based on the speed of the gantry, and determine whether to engage the at least one brake based on the estimated stopping distance.

According to one or more example embodiments, the estimated stopping distance corresponds to a motor stopping distance.

According to one or more example embodiments, a brake comprises braking arms; an electric actuator including a plunger, and a solenoid defining a channel to receive the plunger, the solenoid configured to cause the plunger to move based on a voltage applied to the electric actuator, the electric actuator configured to cause the braking arms to engage a rotor based on a voltage applied to the electric actuator; and a linkage coupled to the plunger and the braking arms, the linkage configured to translate an axial force from the plunger to move the braking arms.

According to one or more example embodiments, a non-transitory computer readable medium stores instructions, when executed by processing circuitry of a radiation therapy machine, cause the radiation therapy machine to obtain a braking signal; estimate a stopping distance of a gantry; determine if the estimated stopping distance is greater than a threshold; and apply a brake if the estimated stopping distance is greater than the threshold.

The present invention also provides a brake for a radiation therapy machine, as defined in claim 1.

Another aspect of the invention provides a brake including braking arms, and an electric actuator, the electric actuator configured to cause the braking arms to engage a rotor based on a voltage applied to the electric actuator. The brake may, by way of example only, be a radiation therapy machine brake. The brake may be used in other applications.

### BRIEF DESCRIPTION OF THE DRAWINGS

The foregoing and other features of the present disclosure will become more fully apparent from the following description and appended claims, taken in conjunction with the accompanying drawings. These drawings depict only several embodiments in accordance with the disclosure and are, therefore, not to be considered limiting of its scope. The disclosure will be described with additional specificity and detail through use of the accompanying drawings.
FIG. 1 schematically illustrates a side view of a radiation therapy system according to one or more example embodiments;
FIG. 2 illustrates a stand coupleable to a gantry frame according to one or more example embodiments;
FIG. 3 illustrates a bearing assembly according to one or more example embodiments;
FIG. 4 illustrates a drive system according to one or more example embodiments;
FIG. 5A illustrates a brake according to one or more example embodiments;
FIG. 5B illustrates another view of the brake shown in FIG. 5A;
FIG. 5C illustrates an exploded view of the brake shown in FIG. 5B;
FIG. 6 illustrates a cross-section of an electric actuator according to one or more example embodiments;
FIG. 7 illustrates a discharge circuit in the electric actuator of FIG. 6 according to one or more example embodiments;
FIG. 8A illustrates a braking arm according to one or more example embodiments;
FIG. 8B illustrates a cross-section of the braking arm shown in FIG. 8A;
FIG. 8C illustrates a braking pad according to one or more example embodiments;
FIG. 9 illustrates an override mechanism being engaged to release the brake pads according to one or more example embodiments;
FIG. 10A illustrates a brake in a released state according to one or more example embodiments;
FIG. 10B illustrates an example embodiment of the brake of FIG. 10A in an engaged state according to one or more example embodiments; and
FIG. 11 illustrates a method of stopping rotation of a gantry according to one or more example embodiments.
FIG. 12 is a block diagram of a control system.

### DETAILED DESCRIPTION

Various example embodiments will now be described more fully with reference to the accompanying drawings in which only some example embodiments are shown. Specific structural and functional details disclosed herein are merely representative for purposes of describing example embodiments. Example embodiments, however, may be embodied in various different forms, and should not be construed as being limited to only the illustrated embodiments. Rather, the illustrated embodiments are provided as examples so that this disclosure will be thorough and complete, and will fully convey the concepts of this disclosure to those skilled in the art. Accordingly, known processes, elements, and techniques, may not be described with respect to some example embodiments. Unless otherwise noted, like reference characters denote like elements throughout the attached drawings and written description, and thus descriptions will not be repeated. The present invention, however, may be embodied in many alternate forms and should not be construed as limited to only the example embodiments set forth herein.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

When the words "about" and "substantially" are used in this application in connection with a numerical value, it is intended that the associated numerical value include a tolerance of ±10% around the stated numerical value, unless otherwise explicitly defined. Further, regardless of whether numerical values are modified as "about" or "substantially," it will be understood that these values should be construed as including a of ±10% around the stated numerical value.

Independent of the grammatical term usage, individuals with male, female or other gender identities are included within the term.

As noted previously, radiation therapy systems commonly employ drive systems that enable precise and repeatable rotational positioning of a radiation source about a treatment couch. As requirements for radiation therapy systems become more stringent, such as higher gantry rotation speeds and lower tolerances for rotational error, operation of clinic gantry axes has been accomplished by limiting the rotation speed and incorporating brakes into a gantry motor to bring the rotation to a stop.

Example embodiments provide a brake with a fast response, permitting a gantry axis on a radiation therapy system to operate at speeds greater than 7 degrees/second. In some example embodiments, the brake delivers large arresting torques to the gantry axis by being integrated outside of a traditional chain based drive system; provides a fail-safe operation by being electrically released with a solenoid and normally engaged with coil springs; applies large arresting torques quickly and consistently by utilizing optimized discharging of the release solenoid; fits within typical available space on a LINAC by utilizing a multi-stage release linkage and a work optimized solenoid; provides consistent operation by allowing brake pads to develop full frictional interfaces even within some variance of a brake rotor position by the design of the multi-stage release linkage; operates within an available power and heat budget of the radiation therapy system by using a strike and hold voltage control; and provides a safe stopping performance by monitoring stops in progress that are using the motor.

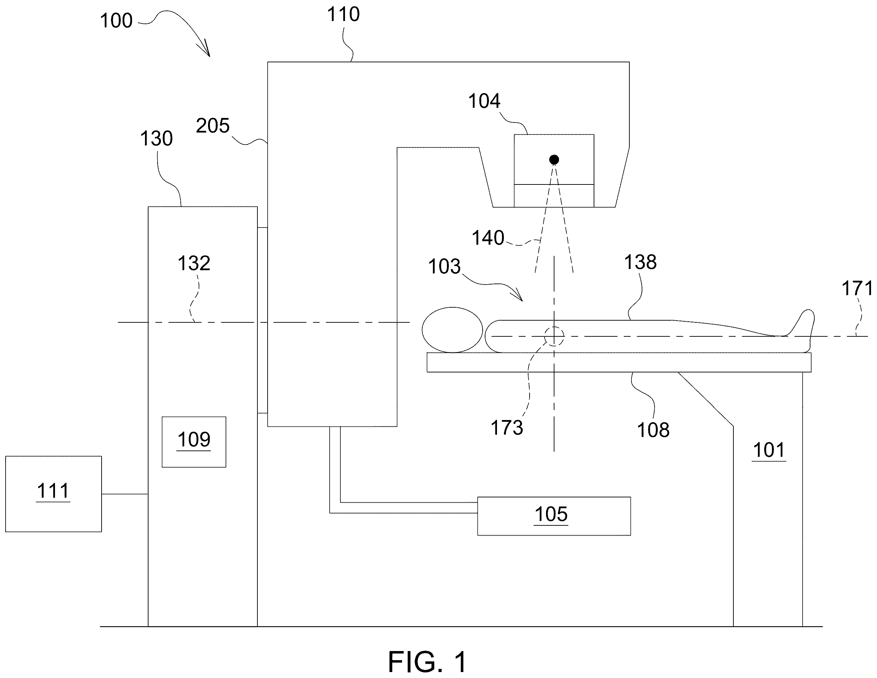

FIG. 1 is a side view of a radiation therapy system 100 according to one or more example embodiments. Radiation therapy (RT) system 100 is a radiation system that may be configured to detect intra-fraction motion in near-real time using either optical or X-ray imaging techniques, or both. Thus, in some embodiments, RT system 100 is configured to provide stereotactic radiosurgery and precision radiotherapy for lesions, tumors, and conditions anywhere in the body where radiation treatment is indicated. As such, RT system 100 can include one or more of a linear accelerator (LINAC) 104 that generates an MV treatment beam of high energy X-rays or other radiation, one or more kilovolt (kV) imaging X-ray sources 106, one or more imaging panels 105. In the embodiment illustrated in FIG. 1, RT system 100 is configured with a gantry 110 capable of rotation about a single axis via a motor and bearing connection.

In some embodiments, RT system 100 is capable of X-ray imaging of a target volume immediately prior to and/or during application of an MV treatment beam, so that an image-guided radiation therapy (IGRT) and/or an intensity-modulated radiation therapy (IMRT) process can be performed using X-ray imaging. For example, in some embodiments, such processes can include kV imaging of the target volume in conjunction with imaging generated by the MV treatment beam. RT system 100 may include one or more touchscreens (not shown) for patient information verification, couch motion controls 102, a radiation area 103, a couch positioning assembly 101, a couch 108 disposed on couch positioning assembly 101, and an image acquisition and treatment control computer 109 (processing circuitry), all of which are disposed within a treatment room. RT system 100 further includes a remote control console 111, which is disposed outside the treatment room and enables treatment delivery and patient monitoring from a remote location. Couch positioning assembly 101 is configured to precisely position couch 108 with respect to radiation area 103. Motion controls include input devices, such as buttons and/or switches, that enable a user to operate couch positioning assembly 101 to automatically and precisely position couch 108 to a predetermined location with respect to radiation area 103. Motion controls 102 also enable a user to manually position couch 108 to a particular location, such as a planned treatment position for a patient or anatomical target.

The RT system 100 includes a base stand 130 and the gantry 110. Base stand 130 is a fixed support structure for components of RT treatment system 100, including the gantry 110 and a drive system (not shown) for rotatably moving the gantry 110 about a horizontal rotation axis 132. Base stand 130 rests on and/or is fixed to a support surface that is external to the RT treatment system 100, such as a floor of an RT treatment facility. The gantry 110 is rotationally coupled to base stand 130, for example via a drive system 205. The gantry 110 is a support structure on which various components of RT system 100 are mounted, including the LINAC 104 and the one or more imaging panels 105.

The LINAC 104 is a radiation source, and typically includes one or more of an electron gun for generating electrons, an accelerating waveguide, an electron beam target, an electron beam transport means (such as a bending magnet) for directing the electron beam to the electron beam target, and/or a collimator assembly 138 for collimating and shaping a treatment beam 140 that originates from the electron beam target. Collimator assembly 138 typically includes one or more of a primary collimator that defines the largest available circular radiation field for treatment beam 140, a secondary collimator for providing a rectangular or square radiation field at isocenter 173 (for example via X-jaws and Y-jaws), and a multileaf collimator (MLC) for conforming treatment beam 140 to a planning target volume (PTV) or another anatomical target. In other embodiments, the LINAC 104 can be any other radiation source suitable for radiation therapy.

During radiation treatment, the LINAC 104 is configured to generate treatment beam 140, which can include high-energy radiation (for example MV X-rays or MV electrons). In other embodiments, treatment beam 140 includes electrons, protons, and/or other heavy charged particles, ultra-high dose rate X-rays (e.g., for FLASH radiotherapy), and/or microbeams for microbeam radiation therapy. Further, in some embodiments, as treatment beam 140 is directed to the isocenter 173 while the gantry 110 rotates through a treatment arc, image acquisitions can be performed via the one or more imaging panels 105 to generate image data for a target volume.

During operation of the RT treatment system 100, the gantry 110 rotates about radiation area 103 when actuated by a drive system for rotatably moving the gantry 110 about horizontal rotation axis 132. Ideally, the isocenter 173 corresponds to the location of a target volume to be treated, such as a PTV, a gross tumor volume (GTV), a clinical target volume (CTV), and/or an internal target volume (ITV), among others. According to various embodiments, a drive and brake system of the RT treatment system 100 enables precise and repeatable rotational positioning of the LINAC 104 (or any other suitable radiation source) about the isocenter 173.

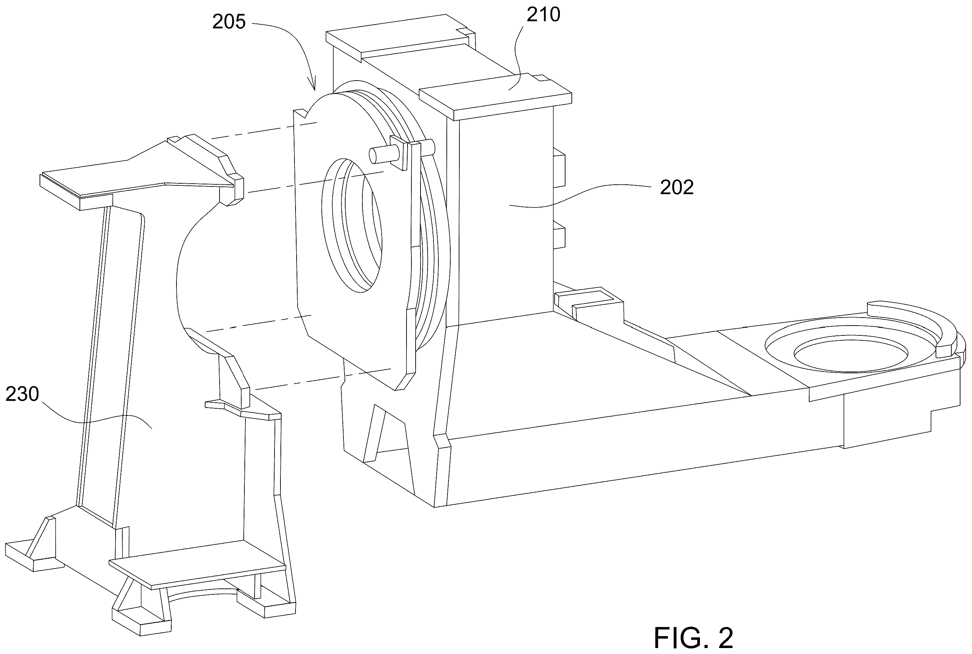

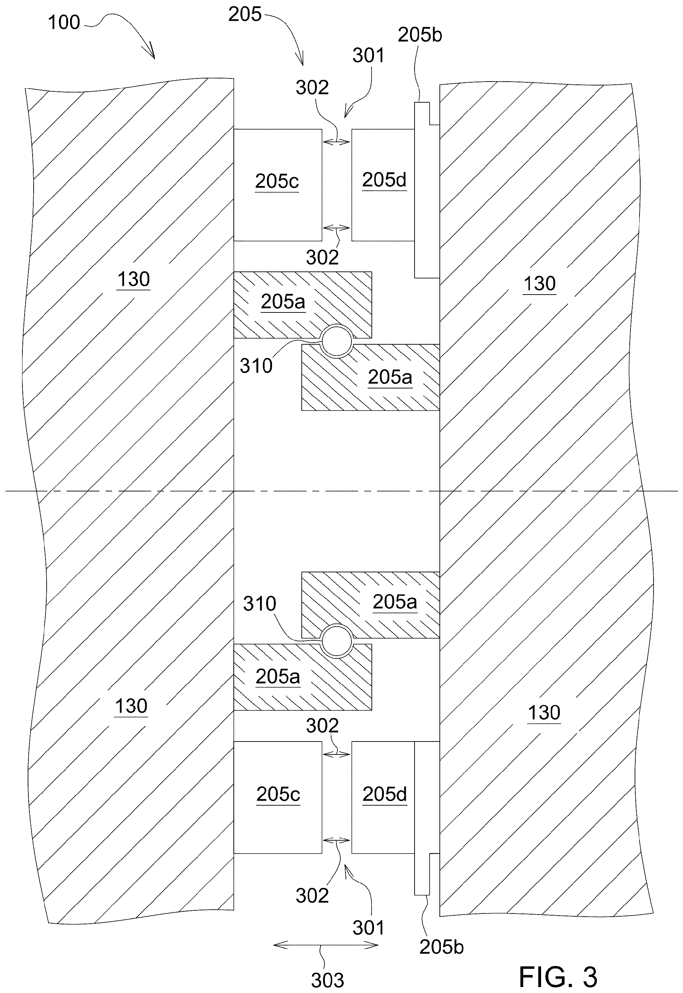

FIG. 2 illustrates a stand 230 (e.g., the stand 130) coupleable to a gantry frame 210 via the drive system 205. FIG. 3 illustrates a more detailed view of the driving system 205 according to one or more example embodiments. As shown in FIG. 3, the drive system 205 may include a bearing plate 205a, a braking rotor 205b, motor coils 205c and motor magnets 205d.

As shown, the C-arm gantry 110 is mechanically coupled to the base stand 130 via the plate bearing 205a, where the bearing plate 205a enables the C-arm gantry 110 to rotate about a horizontal rotation axis 202. According to various embodiments, the rotation of the C-arm gantry 110 about horizontal rotation axis 202 is enabled by the drive system 205.

In one or more example embodiments, the drive system 205 is configured as an axial flux electric motor that includes the coils 205c and the magnets 205d, where the coils 205c are fixed to the base stand 130 and the magnets 205d are fixed to the C-arm gantry 110 via the braking rotor 205b. The braking rotor 205b is fixed to the C-arm gantry 110. As shown, the coils 205c and the magnets 205d are separated from each other by an air gap 301, and therefore the coils 205c and the magnets 205d are not mechanically coupled to each other. The drive system 205 is configured as a direct-drive system, in which there is no interposing drive train between the coils 205c and the magnets 205d.

In the embodiment illustrated in FIG. 3, the magnetic flux 302 in an axial direction 303 (i.e., in a direction parallel to horizontal rotation axis 202) passes to and from the coils 205c and from and to the magnets 205d via the air gap 301. Further, magnetic flux (not shown in FIG. 3) also passes through the coils 205c and the magnets 205d in a direction that is perpendicular to the axial direction 303, which exerts torque about the horizontal rotation axis 202 on the coils 205c and the magnets 205d. Because the coils 205c are fixed to the base stand 130 and the magnets 205d are fixed to the C-arm gantry 110, the torque exerted causes the C-arm gantry 110 to rotate about the horizontal rotation axis 202 via bearings 310 within the bearing plate 205a. Thus, the electric motor of drive system 205 causes rotation of the C-arm gantry 110 with no interposing drive train.

Further description of the drive system 205 and other example embodiments of a drive system are further described in U.S. Patent Application No. 18/882,788, filed Sept. 12, 2024, the entire contents of which are hereby incorporated by reference.

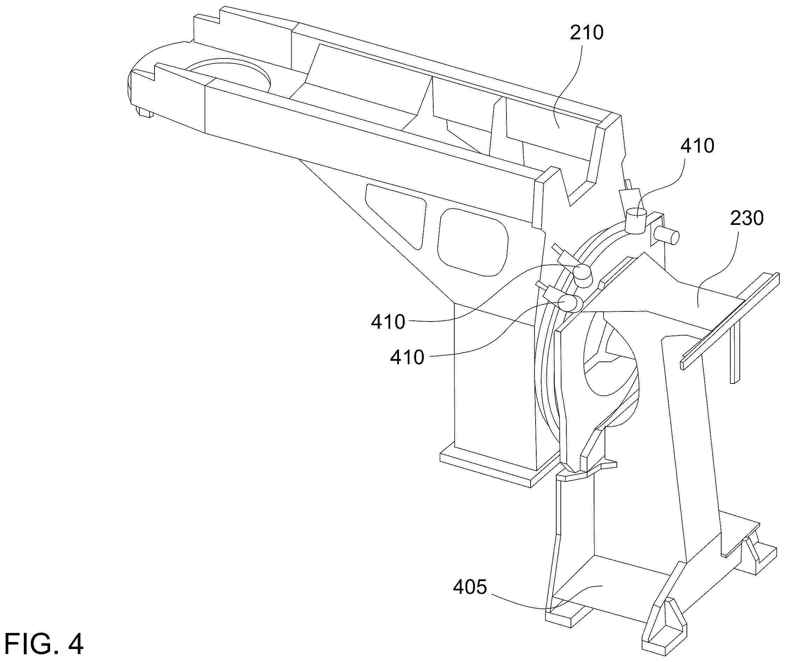

FIG. 4 illustrates one or more example embodiments of a drive system with a brake.

As shown in FIG. 4, the radiation therapy machine may include a plurality of brakes 410 to stop the rotation of the braking rotor 205b and, as a result, stop the rotation of the gantry 110.

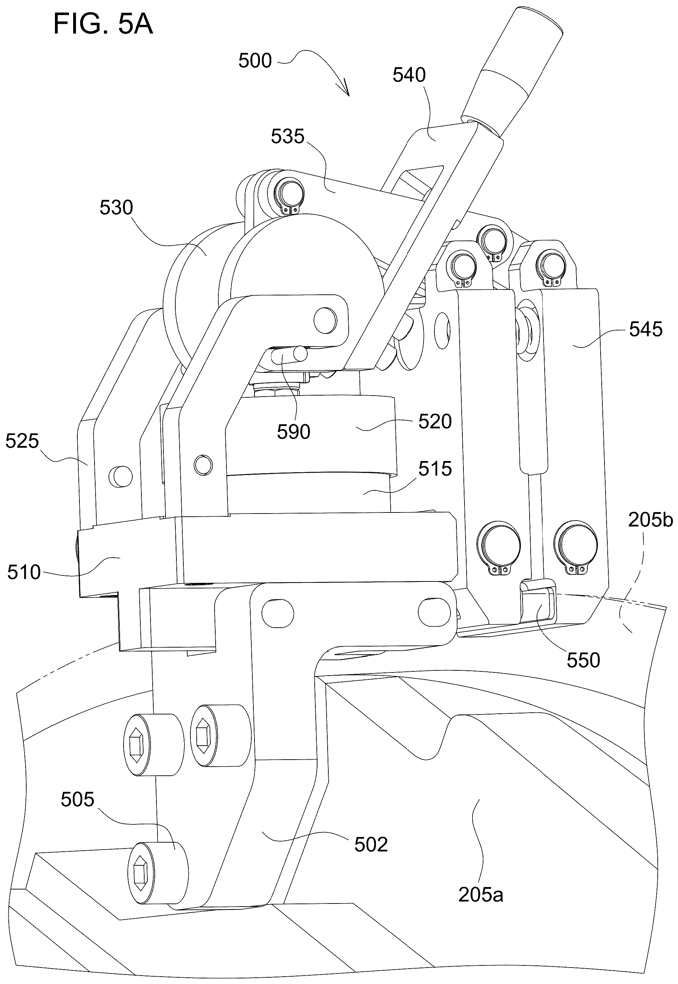

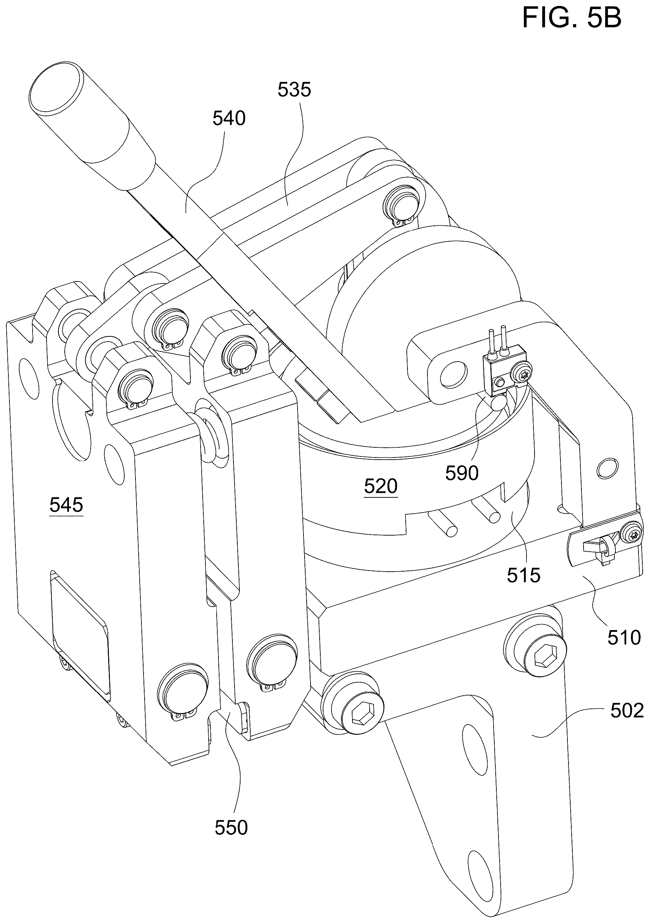

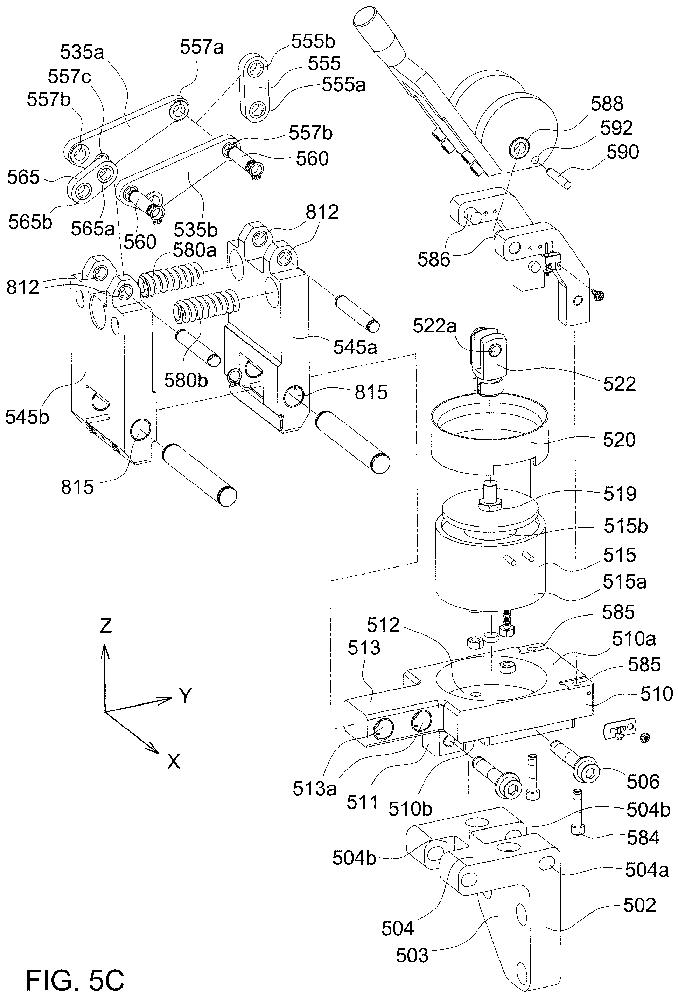

FIG. 5A illustrates a brake according to one or more example embodiments. FIG. 5B illustrates the brake of FIG. 5A from a different view. FIG. 5C illustrates an exploded view of the brake shown in FIG. 5B.

As shown in FIG. 5A, a brake 500 is mounted to the bearing plate 205a of the driving system 205. The brake 500 includes a bracket 502 and may be mounted to the bearing plate 205a of the driving system 205 via the bracket 502 by fasteners 505 such as bolts.

The brake 500 further includes a baseplate 510, an electric actuator 515, finger guard 520, handle supports 525, cams 530, a linkage 535, a handle 540, braking arms 545 and braking pads 550. The braking pads 550 are configured to engage with the braking rotor 205b to stop the driving system 205 from rotating. As will be described, the brake 500 is configured to be part of a fail-safe operation and stop rotation of the gantry if an error or fault occurs such as power loss.

Referring to FIG. 5C, the bracket 502 includes a surface 503 that contacts the bearing plate 205a. The bracket 502 further includes a side 504 that is substantially normal to the surface 503 and provides a cantilever like support to the baseplate 510.

The baseplate 510 includes a protrusion 511. The protrusions 511 includes two apertures that align with apertures 504a of the bracket 502. More specifically, the side further includes to gaps 504b. The protrusions 511 are placed in the gaps 504b, which align the apertures of the protrusions 511 with the apertures 504a. Bolts 506 are used to couple the baseplate 510 to the bracket 502. Each bolt extends through a first aperture 504a, then through an aperture of the protrusion 511 (located in the gap 504b) and then through another aperture 504a.

The baseplate 510 further includes a receiving area 512 a side 510a of the baseplate 510 that opposes a side 510b of the baseplate having the protrusions 511. The baseplate 510 further includes a tab section having two apertures 513a, each of which are used to couple the baseplate 510 to the braking arms 545.

The electric actuator 515 is placed in the receiving area 512. In some example embodiments, the electric actuator 515 includes a solenoid 515a and a plunger 515b. The electric actuator 515 is configured to move a portion of the plunger 515b into a channel of the solenoid 515a when a first voltage is applied to the electric actuator 515 and is configured to maintain the position of the portion of the plunger 515b in the channel when a second voltage is applied to the electric actuator, the second voltage being less than the first voltage.

As shown in FIGS. 5A and 5B, only a portion of the solenoid 515a is placed in the receiving area 512 to limit movement along the x-y directions.

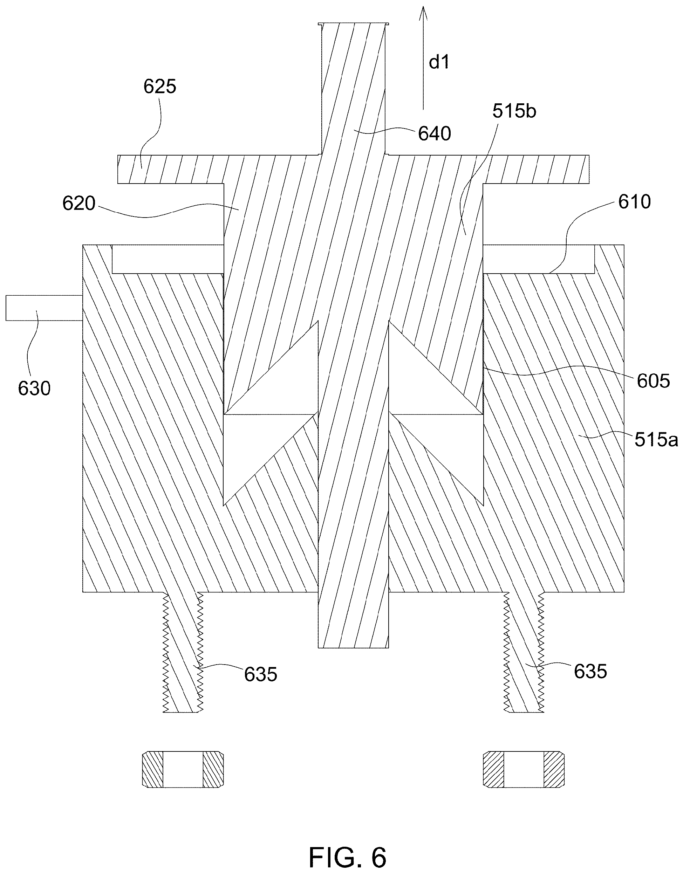

FIG. 6 illustrates a cross-sectional drawing of the electric actuator 515. As shown, the solenoid 515a includes a channel 605 extending partially through the solenoid 515a along a longitudinal axis of the solenoid 515a. The channel 605 receives the plunger 515b. The plunger 515b includes a cylindrical body 620 and a top 625 at an end of the cylindrical body 620. The top 625 has an outer diameter greater than an outer diameter of the cylindrical body 620. When the plunger 615 is engaged in the channel 605, the top 625 rest in a receiving area 610 of the solenoid 515a above the channel 605.

Leads 630 allow the controller 109 to apply a voltage to the electric actuator 515 via the leads 630. The solenoid 515a may be a low resistance solenoid to allow for a high power operation on a voltage limited power architecture. In some example embodiments, the solenoid 515a develops at least 500N of pull out force at 2.2 A at 0 mm stroke. Based on the voltage applied to the electric actuator, the solenoid 515a is configured to cause the plunger to move. For example, during a power failure (i.e., 0 V or close to 0 V), at least a portion of the plunger 515b moves along the longitudinal direction of the solenoid 515a such that the portion moves out of the channel 605 and the top 625 moves out of the receiving area (along a direction d1). The movement along the direction d1 causes the braking arms 545 to engage the braking rotor 205b and stop rotation of the gantry 110.

The electric actuator 515 may further include a discharge circuit to decay recirculation current and tune the motion of the plunger 515b releasing (e.g., to reduce/avoid damage and/or to reduce/avoid noise due to brake engagement).

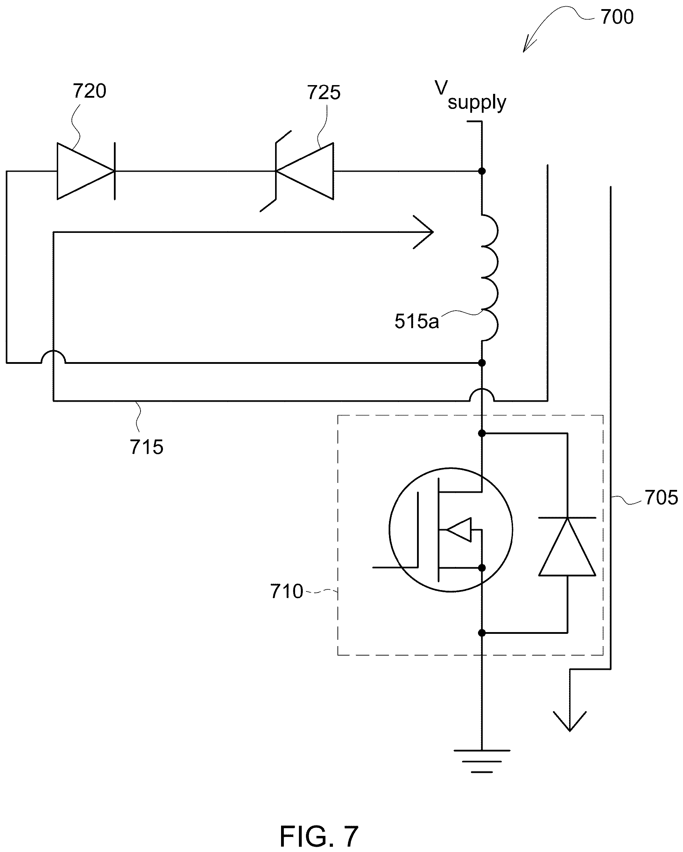

FIG. 7 illustrates a discharge circuit 700 in the electric actuator 515 according to one or more example embodiments. During normal operation of the gantry rotating (brake 500 is released), a voltage Vsupply is supplied from the controller 109 and a drive current 705 passes through the solenoid 515a and a switching 710 that is on. A recirculation current 715 exists. To control the decay rate of the recirculation current 715 (and the voltage across the inductor), a diode 720 and a Zener diode 725 are placed in series across the solenoid 515a. The Zener diode 725 slows down the engagement of the brake 500 (relative to a circuit without a Zener diode) such that mechanical shock is mitigated while maintaining a sufficient brake application speed and brake arresting force. The Zener diode 725 provides a slowdown in the decay of the recirculation current 715, which reduces the mechanical shock caused by the brake 500 engaging the braking rotor 205b (e.g., due to kinetic energy provided by coil springs 580a, 580b, which are discussed further below) and reduces the noise caused by the braking engagement. The Zener diode 725 can be selected to have a particular voltage based on an application for the discharge circuit and characteristics associated with the application (e.g., force requirements, speed requirements, and noise requirements). In an example embodiment, the Zener diode 725 may be a 24V Zener diode.

More specifically, a first end of the solenoid is coupled to an anode of the diode 720 and a second end of the solenoid is coupled an anode of the Zener diode 725.

Referring back to FIG. 6, the electric actuator 515 further includes fasteners 635 to couple the electric actuator 515 to the receiving area 512. In some example embodiments, the fasteners 635 may be threaded screws and nuts. The fasteners 635 may extend through apertures in the receiving area 512 and the nuts may be used to screw onto the screws and couple the electric actuator 515 to the receiving area 512.

The plunger 515b may further include a threaded protrusion 640 to couple the electric actuator 515 to a clevis 522, shown in FIG. 5C. More specifically, the threaded protrusion 640 is sued to translate the force from the plunger 515b to the clevis 522.

As shown in FIG. 5C, the clevis 522 is attached to the threaded protrusion 640 at one end and is pivotally attached to a link 555 at an opposing end. The link 555 minimizes/reduces non-axial forces on the solenoid 515a.

A nut 519 is on the threaded protrusion 640 between the clevis 522 and the plunger 515b. The nut 519 prevents unintended motion (e.g., along the z-axis) between the clevis 522 and the plunger 515b. The use of the nut 519 and the threaded protrusion 640 allows a distance between the clevis 522 and the plunger 515b to be adjustable.

The clevis 522 includes a U-shaped gap (or rectangular-shaped gap) with an aperture 522a at each end of the U-shaped gap. The link 555 has curved ends with substantially straight sides between the curved ends. The link 555 further includes apertures 555a and 555b at each of the curved ends. The link 555 is placed within the U-shaped gap of the clevis 522 such that the apertures 522a align with the aperture 555a. A pin may be inserted through the apertures 522a and the aperture 555a to permit the link 555 to rotate about a concentric central axis of the apertures 522a and the aperture 555a.

The link 555 is also coupled to the linkage 535 via aperture 555b. The linkage 535 includes two triangle-like shaped linkage arms 535a and 535b. Each linkage arm 535a, 535b includes an aperture 557a at a first end and two apertures 557b, 557c at a second end. A width of the linkage arms 535a, 535b tapers from the second end to the first end. The clevis is placed between the linkage arms 535a, 535b such that the aperture 555b aligns with the apertures 557a. A shaft 560 is inserted through the apertures 557a and the aperture 555b to couple the link 555 with the linkage arms 535a, 535b.

Another link 565 is coupled to the linkage arms 535a, 535b via the apertures 557b. The link 565 has curved ends with substantially straight sides between the curved ends. The link 565 further includes apertures 565a and 555b at each of the curved ends. The link 565 is placed between the linkage arms 535a, 535b such that the apertures 557b align with the aperture 565a. Another shaft 560 may be inserted through the apertures 557b and the aperture 565a to permit the link 565 to rotate about a concentric central axis of the apertures 557b and the aperture 565a.

The linkage arms 535a, 535b are also directly coupled to one of the braking arms 545.

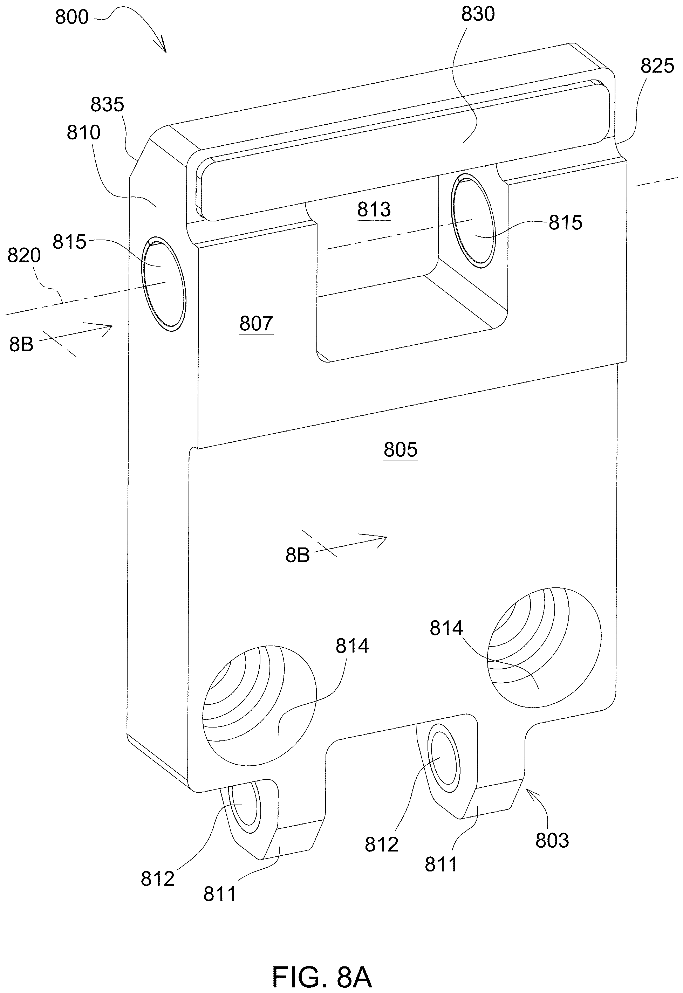

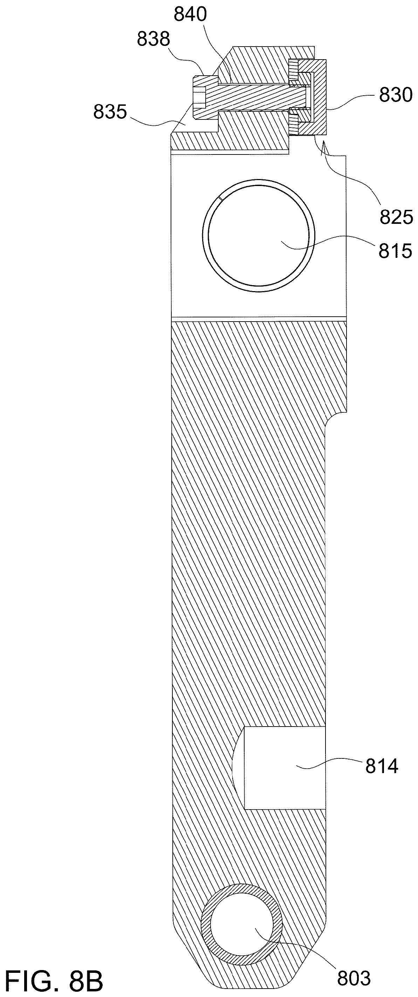

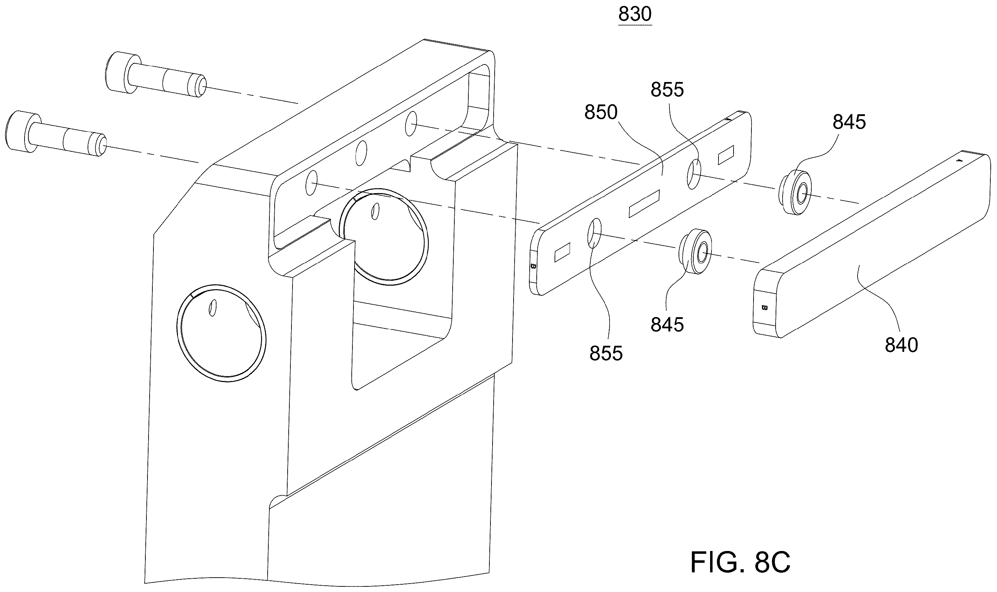

FIG. 8A illustrates a braking arm according to one or more example embodiments. FIG. 8B illustrates a cross section of the braking arm shown in FIG. 8A and FIG. 8C illustrates a braking pad according to one or more example embodiments.

As shown in FIGS. 8A and 8B, a braking arm 800 includes a linkage coupling section 803, a rectangular body 805, a baseplate coupling section 807 and a brake pad receiving section 810. The linkage coupling section 803 includes two semi-circular protrusions 811 from the rectangular body. Each protrusion includes an aperture 812 therethrough. The rectangular body 805 includes two counterbores 814 with each counterbore 814 near the protrusions 811. The baseplate coupling section 807 is on a side of the rectangular body 805 that opposes a side of the rectangular body 805 having the counterbores 814. The baseplate coupling section 807 defines a rectangular-shaped gap 813. The baseplate coupling section 807 further includes apertures 815 such that a concentric central axis 820 of the apertures 815, extends through one aperture 815, across the rectangular-shaped gap 813 and through the other aperture 815.

The brake pad receiving section 810 is on a side of the baseplate coupling section 807 that opposes a side of the baseplate coupling section 807 on the rectangular body 805. The brake pad receiving section 810 includes a pocket 825 that is indented relative to the baseplate coupling section 807 to constrain a brake pad 830. The brake pad receiving section 810 further includes a fastener receiving are 835 on a side of the brake pad receiving section 810 that opposes a side on which the brake pad 830 is mounted. The brake pad 830 is mounted to the brake pad receiving section 810 via fasteners 838 (e.g., threaded fasteners) extending through an aperture 840 of the brake pad receiving section 810. The fasteners 838 permit the brake pads 830 to be replaced for service. The fasteners 838 may use precote^{®} for vibration resistance.

FIG. 8C illustrates a brake pad 830 according to one or more example embodiments. The brake pad 830 may include a friction material 840, nuts 845 and a backing 850. The backing 850 may be made of steel. As shown, the backing 850 includes apertures 855 to receive the fasteners 838. The nuts 845 as adhered to both the friction material 840 and the backing 850. The fasteners 838 are inserted into the nuts 845 to couple the brake pads 830 to the braking arms 800.

Referring back to FIG. 5C, the braking arms 545 include braking arms 545a and 545b. Each of the braking arms 545a, 545b may be the same as the braking arm 800.

The braking arm 545b is coupled to the link 565. More specifically, the link 565 is placed between the protrusions (e.g., 811) of the braking arm 545b such that the apertures 812 of the braking arm 545b align with the aperture 565b. A shaft is inserted through the apertures 812 of the braking arm 545b and the aperture 565b.

The braking arm 545a is coupled to the linkage arms 535a, 535b. More specifically, the linkage arms 535a, 535b are placed between the protrusions (e.g., 811) of the braking arm 545a such that the apertures 812 of the braking arm 545a align with the apertures 557c. A shaft is inserted through the apertures 812 of the braking arm 545a and the apertures 557c.

The braking arms 545a, 545b are also connected to the mounting baseplate 510. More specifically, a tab portion 513 is placed in the rectangular-shaped gap (e.g., 813) of both braking arms 545a, 545b. One shaft is inserted into an aperture 513a that aligns with the apertures 815 of the braking arm 545a and another shaft is inserted into the other aperture 513a that aligns with the apertures 815 of the braking arm 545b. The shafts are also inserted through the respective pair of apertures 815 to allow rotation about a central axis of the shaft.

Coil springs 580a, 580b provide a clamping force to allow for a fail-safe braking operation. The coil spring 580a is placed into a counterbore 814 of the braking arm 545a and a counterbore 814 on the same y-axis of the braking arm 545b. The coil spring 580b is placed into the other counterbore 814 of the braking arm 545a and the other counterbore 814 of the braking arm 545b.

Due to the arrangement of the clevis 522, the link 555, the linkage arms 535a, 535b and the link 565, a linkage is provided that is configured to translate an axial force from the plunger 515b (e.g., in the positive z direction) to move the braking arms 545a, 545b.

The handle supports 525, the cams 530 and the handle 540 form an override mechanism that causes the braking arms to disengage the braking rotor 205b independent of the voltage applied to the electric actuator 515. Each of the handle supports 525 are C-c\shaped with first ends being coupled to the mounting baseplate 510 using fasteners 584 that extend through apertures 585 of the mounting baseplate 510 and into the first ends of the handle supports 525. Second ends of the handle support 525 include protrusions 586 extending into spacing between the handle supports 525. The cams 530 include center apertures 588. The cams 530 are attached to the handle supports 525, respectively, by inserting the respective protrusion into the aperture 588.

A magnetic dowel pin 590 is placed into an aperture 592 of the cams 530. The magnetic dowel pin 590 allows for magnetic retention of the handle 540 in a normal position (see, FIG. 5A), to provide a hard stop for the handle 540 and to actuate a handle position switch 960.

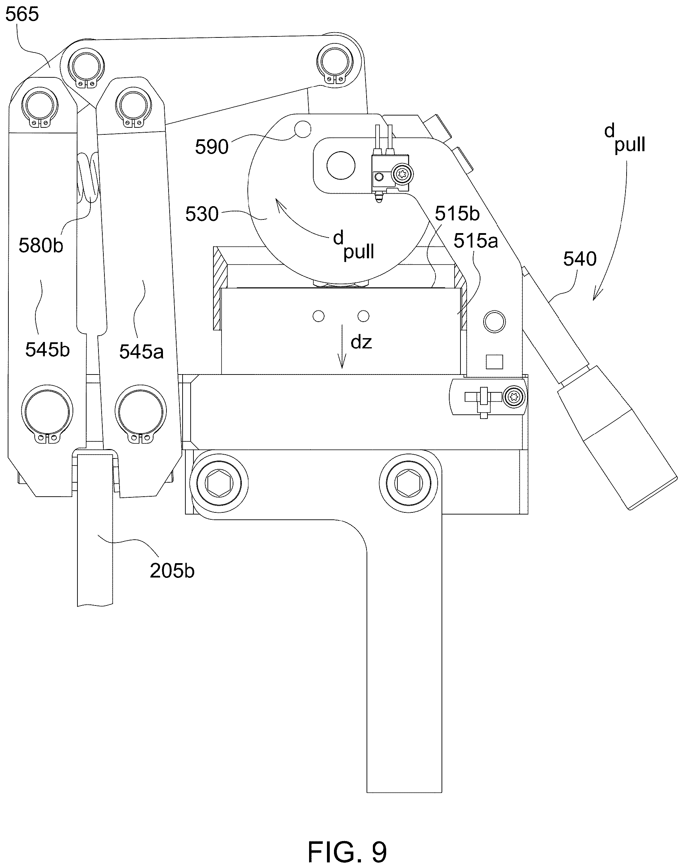

FIG. 9 illustrates an example embodiment of the override mechanism being engaged to release the brake pads from the braking rotor 205b. The example of FIG. 9 omits the fingerguard 520 for clarity. In the example of FIG. 9, the handle 540 is pulled in the direction dpull. This causes the cams 530 to rotate in the same direction which causes the cams 530 to apply an axial force in the direction dz. The axial force in the dz direction causes the plunger 515b to be inserted into the solenoid 515a. In addition, the linkage (e.g., the clevis 522, the link 555, the linkage arms 535a, 535b and the link 565) translate this axial force into a compression force on the coil springs 580b, 580a, which causes the braking arms 545a, 545b to release the braking rotor 205b.

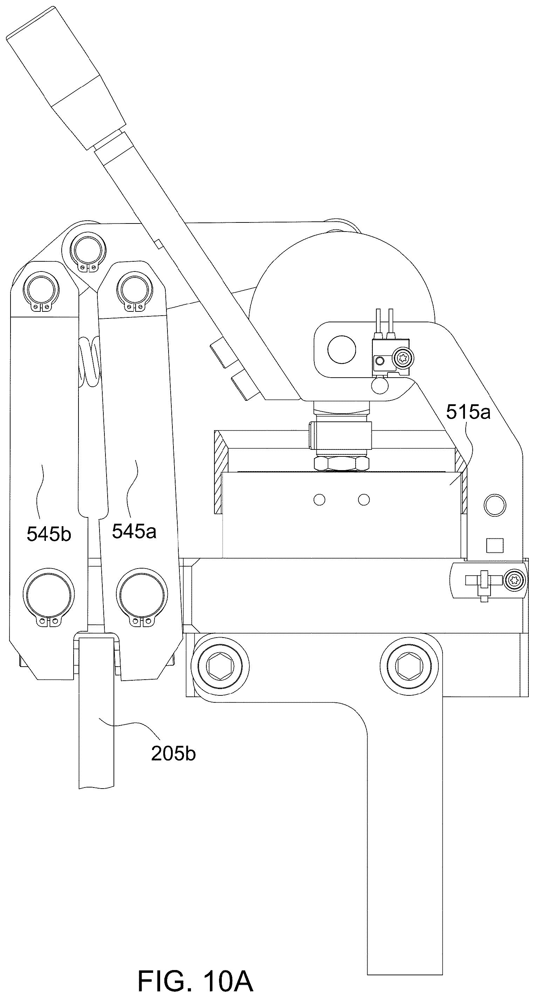

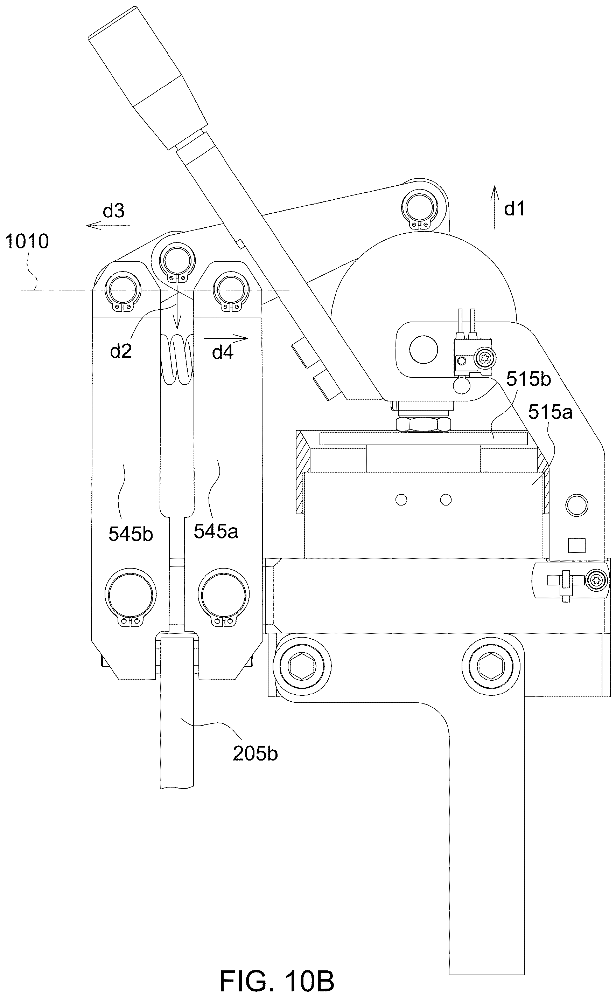

FIG. 10A illustrates an example embodiment of the brake 500 in a released state. FIG. 10B illustrates an example embodiment of the brake 500 in an engaged state. The examples of FIGS. 10A-10B omit the fingerguard 520 for clarity. As shown in FIG. 10A, when the plunger 515b (including the top) is in the solenoid 515a, the brake is released from the braking rotor 205b. As shown in FIG. 10B, when a portion of the plunger 515b has moved in the positive z-direction (d1) and become magnetically disengaged from the solenoid 515a (e.g., due to power loss), the braking arms 545a, 545b engage the braking rotor 205b and cause the gantry to stop rotating and/or prevent the gantry from rotating. More specifically, the linkage translates movement of the plunger in the positive z-direction to the shaft 560 to move in the negative z-direction (d2) which causes the upper portion of the braking arms (e.g., at 812) to move along an axis 1010 normal to the z-axis. The upper portion of the braking arm 545b moves into a direction d3 and the upper portion of the braking arm into a direction d4 which is opposite of the direction d3.

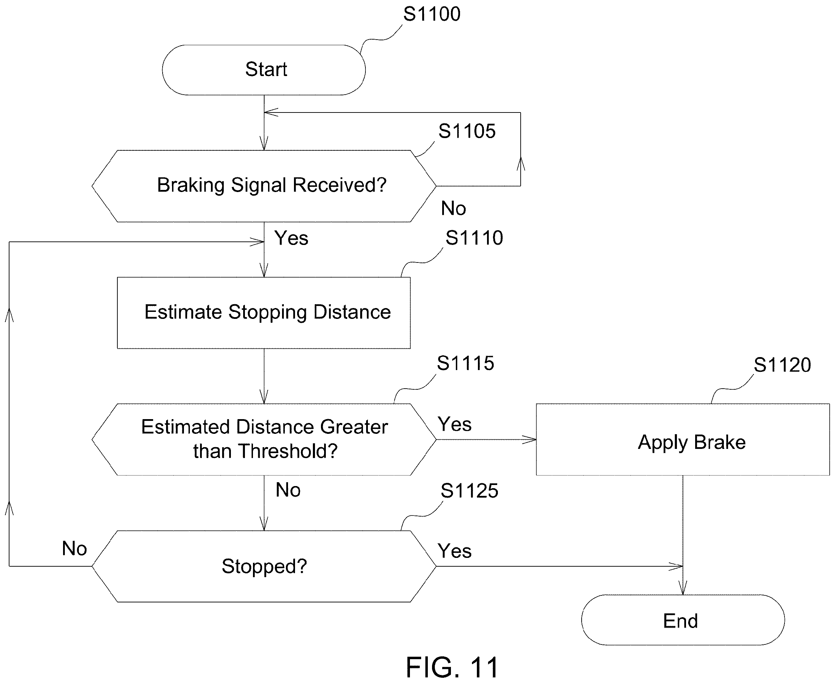

FIG. 11 illustrates a method of stopping rotation of a gantry according to one or more example embodiments. The method in FIG. 11 may be performed by the system 100. More specifically, the treatment control computer 109 may be configured to cause the system 100 to perform the method of FIG. 11 (e.g., by executing instructions stored in memory).

At S1100, the method starts. At S1105, the treatment control computer 109 monitors to whether a braking signal is received by the system. For example, the console 111 may issue a braking signal for the gantry to stop rotating and or the braking of the rotation may be predetermined based on a treatment therapy or a possible collision.

If a braking signal is received, the treatment control computer 109 may estimate a stopping distance of the gantry at S1110.

In some instances the gantry is stopped only using the motor. More specifically, the treatment control computer 109 may attempt to stop the gantry rotation without actuating the brakes unless there is reason to believe that a collision may be imminent or that there is an issue within the motor drive system.

Rules and/or regulations may require that the gantry stop rotation within a particular threshold distance. In some example embodiments, the threshold distance may be 3 degrees. To prevent or the reduce the likelihood of a stopping distance of the gantry exceeding the threshold distance, the treatment control computer executes a motor stop monitoring algorithm such that the gantry comes to a stop within a safe stopping distance by measuring the velocity of the gantry after a braking signal is received and estimating what the stopping distance would be if the brakes were applied.

The brake-only stopping distance is estimated by the treatment control computer 109 using a two term function: $Y = .03 ∗ V + 0.01 ∗ {V}^{2} + X$ where Y is the estimated brake-only stopping distance (in degrees) from when the motor-only stop was commanded, V is the axis velocity (in degrees per second) and X is the distance that has already elapsed (distance gantry has moved) since the motor-only stop was commanded (the braking signal).

At S1115, the treatment control computer 109 determines whether the estimated brake-only stopping distance is greater than the threshold distance. If the estimated brake-only stopping distance exceeds the threshold distance, the brakes will be applied by the treatment control computer 109 at S1120.

If the estimated brake-only stopping distance is lower than or equal to the threshold distance, the method proceeds to S1125 to determine whether the gantry has stopped rotating. If the gantry continues to rotate (e.g., measured by a speed sensor), the method returns to S1110.

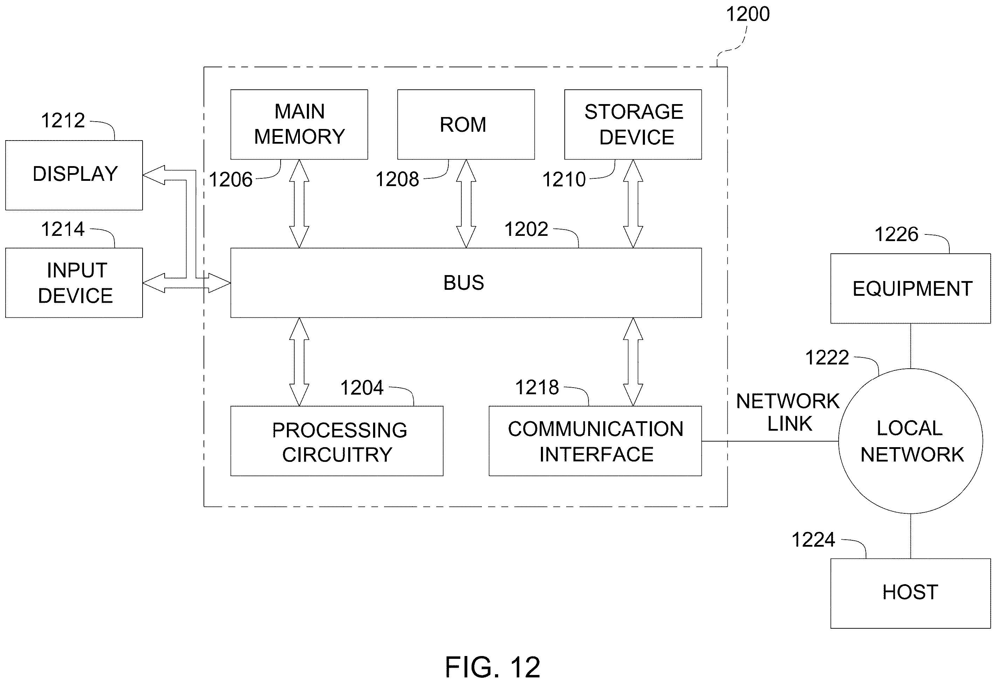

FIG. 12 is a block diagram illustrating an embodiment of a specialized control system 1200 that can be used to implement various embodiments described herein. For example, the control system 1200 may be configured to control the brake in accordance with example embodiments.

Also, in some embodiments, the control system 1200 may be used to implement the processing circuitry 109 and/or the remote control console 111. The control system 1200 may also be an example of any control system described herein.

The control system 1200 includes a bus 1202 or other communication mechanism for communicating information, and processing circuitry 1204 (e.g., at least one processor and/or ASIC) coupled with the bus 1202 for processing information. In examples where the processing circuitry 1204 is hardware configured to executed stored instructions (e.g., a processor), the control system 1200 also includes a main memory 1206, such as a random-access memory (RAM) or other dynamic storage device, coupled to the bus 1202 for storing information and instructions to be executed by the processing circuitry 1204. The main memory 1206 also may be used for storing temporary variables or other intermediate information during execution of instructions to be executed by the processing circuitry 1204. The control system 1200 further includes a read only memory (ROM) 1208 or other static storage device coupled to the bus 1202 for storing static information and instructions for the processing circuitry 1204. A data storage device 1210, such as a magnetic disk or optical disk, may be provided and coupled to the bus 1202 for storing information and instructions.

The control system 1200 may be coupled via the bus 1202 to a display 1212, such as a flat panel, for displaying information to a user. An input/output device 1214, such as a touchscreen, is coupled to the bus 1202 for communicating information and command selections to processing circuitry 1204. Another type of user input device is cursor control 1214, such as a mouse, a trackball, or cursor direction keys for communicating direction information and command selections to processing circuitry 1204 and for controlling cursor movement on display 1212. This input device typically has two degrees of freedom in two axes, a first axis (e.g., x) and a second axis (e.g., y), that allows the device to specify positions in a plane.

While the display 1212 and I/O device 1214 are shown outside of the control system 1200, it should be understood that the display 1212 and the I/O device 1214 are part of the control system 1200 such as shown in FIG. 12.

In some embodiments, the control system 1200 can be used to perform various functions described herein such as the method of FIG. 11. According to some embodiments, such use is provided by control system 1200 in response to the processing circuitry 1204 executing one or more sequences of one or more instructions contained in the main memory 1206. The instructions may include instructions to cause a radiation therapy machine to perform the method of FIG. 11. Those skilled in the art will know how to prepare such instructions based on the functions, algorithms and methods described herein. Such instructions may be read into the main memory 1206 from another processor-readable medium, such as storage device 1210. Execution of the sequences of instructions contained in the main memory 1206 causes the processing circuitry 1204 to perform the process steps described herein. One or more processors in a multi-processing arrangement may also be employed to execute the sequences of instructions contained in the main memory 1206. In alternative embodiments, hard-wired circuitry may be used in place of or in combination with software instructions to implement the various embodiments described herein. Thus, embodiments are not limited to any specific combination of hardware circuitry and software.

Transmission media includes coaxial cables, copper wire and fiber optics, including the wires that comprise the bus 1202. Transmission media can also take the form of acoustic or light waves, such as those generated during radio wave and infrared data communications.

Various forms of processor-readable media may be involved in carrying one or more sequences of one or more instructions to the processing circuitry 1204 for execution. For example, the instructions may initially be carried on a magnetic disk of a remote computer. The remote computer can load the instructions into its dynamic memory and send the instructions over a network, such as the Internet or a local network. A receiving unit local to the control system 1200 can receive the data from the network and provide the data on the bus 1202. The bus 1202 carries the data to the main memory 1206, from which the processing circuitry 1204 retrieves and executes the instructions. The instructions received by the main memory 1206 may optionally be stored on the storage device 1210 either before or after execution by the processing circuitry 1204.

The control system 1200 also includes a communication interface 1218 coupled to the bus 1202. The communication interface 1218 provides a two-way data communication coupling to a network link 1220 that is connected to a local network 1222. For example, the communication interface 1218 may be an integrated services digital network (ISDN) card or a modem to provide a data communication connection to a corresponding type of telephone line. As another example, the communication interface 1218 may be a local area network (LAN) card to provide a data communication connection to a compatible LAN. Wireless links may also be implemented. In any such implementation, the communication interface 1218 sends and receives electrical, electromagnetic or optical signals that carry data streams representing various types of information.

The network link 1220 typically provides data communication through one or more networks to other devices. For example, the network link 1220 may provide a connection through local network 1222 to a host computer 1224 or to equipment 1226 such as a radiation beam source or a switch operatively coupled to a radiation beam source. The data streams transported over the network link 1220 can comprise electrical, electromagnetic or optical signals. The signals through the various networks and the signals on the network link 1220 and through the communication interface 1218, which carry data to and from the control system 1200, are exemplary forms of carrier waves transporting the information. The control system 1200 can send messages and receive data, including program code, through the network(s), the network link 1220, and the communication interface 1218.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments will be described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Central Processing Units (CPUs), Graphical Processing Units (GPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, medical systems, may be (or include) hardware, firmware, hardware executing software or any combination thereof. Such hardware may include processing or control circuitry such as, but not limited to, one or more processors (CPUs), one or more GPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

### NON-LIMITING ILLUSTRATIVE EMBODIMENTS

The following is a list of non-limiting illustrative embodiments disclosed herein:
Illustrative embodiment 1 includes a radiation therapy machine comprising a gantry rotatable about an axis; a rotor coupled to the gantry; and at least one brake, the at least one brake including, braking arms, and an electric actuator, the electric actuator configured to cause the braking arms to engage the rotor based on a voltage applied to the electric actuator.
Illustrative embodiment 2 includes the radiation therapy machine of illustrative embodiment 1, wherein the electric actuator includes a plunger, and a solenoid defining a channel to receive the plunger, the solenoid configured to cause the plunger to move based on the voltage applied to the electric actuator.
Illustrative embodiment 3 includes the radiation therapy machine of illustrative embodiment 1 or 2, wherein the electric actuator is configured to move a portion of the plunger into the channel when a first voltage is applied to the electric actuator and is configured to maintain the position of the portion plunger in the channel when a second voltage is applied to the electric actuator, the second voltage being less than the first voltage.
Illustrative embodiment 4 includes the radiation therapy machine of any one of illustrative embodiments 1-3, wherein the electric actuator further includes, a discharging circuit configured to discharge the voltage applied to the electric actuator, the discharging circuit including a Zener diode.
Illustrative embodiment 5 includes the radiation therapy machine of any one of illustrative embodiments 1-4, wherein the at least one brake further includes, a linkage coupled to the plunger and the braking arms, the linkage configured to translate an axial force from the plunger to move the braking arms.
Illustrative embodiment 6 includes the radiation therapy machine of any one of illustrative embodiments 1-5, wherein the linkage is configured to translate a first axial force in a first direction into a second axial force in a second direction, the first axial force and the second axial force occurring at separate locations.
Illustrative embodiment 7 includes the radiation therapy machine of any one of illustrative embodiments 1-7, wherein the linkage includes a clevis, and the at least one brake further includes a nut between the clevis and the plunger.
Illustrative embodiment 8 includes the radiation therapy machine of any one of illustrative embodiments 1-7, wherein the nut prevents motion of the clevis in a first direction relative to the plunger.
Illustrative embodiment 9 includes the radiation therapy machine of any one of illustrative embodiments 1-8, wherein the linkage includes a clevis, and the at least one brake further includes a shaft, the clevis and the plunger coupled to the shaft such that a distance between the clevis and the plunger is adjustable.
Illustrative embodiment 10 includes the radiation therapy machine of any one of illustrative embodiments 1-9, wherein the at least one brake further includes, at least one spring between the braking arms, the at least one spring configured to provide a clamping force to the braking arms.
Illustrative embodiment 11 includes the radiation therapy machine of any one of illustrative embodiments 1-10, wherein the at least one brake further includes, an override mechanism configured to cause the braking arms to disengage the rotor independent of the voltage applied to the electric actuator.
Illustrative embodiment 12 includes the radiation therapy machine of any one of illustrative embodiments 1-11, wherein the rotor is between the braking arms.
Illustrative embodiment 13 includes the radiation therapy machine of any one of illustrative embodiments 1-12, wherein the electric actuator causes the braking arms to engage the rotor when the voltage applied to the electric actuator is zero volts.
Illustrative embodiment 14 includes the radiation therapy machine of any one of illustrative embodiments 1-13, further comprising a stand; and a drive system, the drive system coupling the stand to the gantry, the at least one brake being mounted to the drive system.
Illustrative embodiment 15 includes the radiation therapy machine of any one of illustrative embodiments 1-14, wherein the drive system includes a bearing assembly coupled to the gantry and the stand, the at least one brake being mounted to a portion of the bearing assembly.
Illustrative embodiment 15 includes the radiation therapy machine of any one of illustrative embodiments 1-15, wherein the at least one brake includes a plurality of brakes arranged around the rotor.
Illustrative embodiment 17 includes the radiation therapy machine of any one of illustrative embodiments 1-16, further comprising processing circuitry configured to cause the radiation therapy machine to, obtain a speed of the gantry, estimate a stopping distance based on the speed of the gantry, and determine whether to engage the at least one brake based on the estimated stopping distance.
Illustrative embodiment 18 includes the radiation therapy machine of any one of illustrative embodiments 1-17, wherein the estimated stopping distance corresponds to a motor stopping distance.
Illustrative embodiment 19 includes a brake comprising braking arms; an electric actuator including a plunger, and a solenoid defining a channel to receive the plunger, the solenoid configured to cause the plunger to move based on a voltage applied to the electric actuator, the electric actuator configured to cause the braking arms to engage a rotor based on a voltage applied to the electric actuator; and a linkage coupled to the plunger and the braking arms, the linkage configured to translate an axial force from the plunger to move the braking arms.
Illustrative embodiment 20 includes the brake of illustrative embodiment 19, wherein the electric actuator is configured to move a portion of the plunger into the channel when a first voltage is applied to the electric actuator and is configured to maintain the position of the portion plunger in the channel when a second voltage is applied to the electric actuator, the second voltage being less than the first voltage.
Illustrative embodiment 21 includes the brake of any one of illustrative embodiments 19-20, wherein the electric actuator further includes, a discharging circuit configured to discharge the voltage applied to the electric actuator, the discharging circuit including a Zener diode.
Illustrative embodiment 22 includes the brake of any one of illustrative embodiments 19-21, wherein the linkage is configured to translate a first axial force in a first direction into a second axial force in a second direction, the first axial force and the second axial force occurring at separate locations.
Illustrative embodiment 23 includes brake of any one of illustrative embodiments 19-22, wherein the linkage includes a clevis, and the brake further includes a nut between the clevis and the plunger.
Illustrative embodiment 24 includes the brake of illustrative embodiment 23, wherein the nut prevents motion of the clevis in a first direction relative to the plunger.
Illustrative embodiment 25 includes the brake of any one of illustrative embodiments 19-24, wherein the linkage includes a clevis, and the brake further includes a shaft, the clevis and the plunger coupled to the shaft such that a distance between the clevis and the plunger is adjustable.
Illustrative embodiment 26 includes the brake of any one of illustrative embodiments 19-25, wherein the brake further includes, at least one spring between the braking arms, the at least one spring configured to provide a clamping force to the braking arms.
Illustrative embodiment 27 includes the brake of any one of illustrative embodiments 19-26, wherein the at least one brake further includes, an override mechanism configured to cause the braking arms to disengage the rotor independent of the voltage applied to the electric actuator.
Illustrative embodiment 28 includes the brake of any one of illustrative embodiments 19-27, wherein the rotor is between the braking arms.
Illustrative embodiment 29 includes the brake of any one of illustrative embodiments 19-27, wherein the electric actuator causes the braking arms to engage the rotor when the voltage applied to the electric actuator is zero volts.
Illustrative embodiment 30 includes a non-transitory computer readable medium storing instructions, when executed by processing circuitry of a radiation therapy machine, cause the radiation therapy machine to obtain a braking signal; estimate a stopping distance of a gantry; determine if the estimated stopping distance is greater than a threshold; and apply a brake if the estimated stopping distance is greater than the threshold.
Illustrative embodiment 31 includes the non-transitory computer readable medium of illustrative embodiment 30, wherein the estimated stopping distance corresponds to a motor stopping distance.
Illustrative embodiment 32 includes the non-transitory computer readable medium of any one of illustrative embodiments 30-31, wherein the braking signal is obtained from a remote console.
Illustrative embodiment 33 includes the non-transitory computer readable medium of any one of illustrative embodiments 30-32, wherein the braking signal is based on a treatment therapy or a possible collision.
Illustrative embodiment 34 includes the non-transitory computer readable medium of any one of illustrative embodiments 30-33, wherein the non-transitory computer readable medium stores the instructions, when executed by the processing circuitry, cause the radiation therapy machine to estimate the stopping distance based on a velocity of the gantry and a distance the gantry has moved since the braking signal was obtained.

While various aspects and embodiments have been disclosed herein, other aspects and embodiments will be apparent to those skilled in the art without departing from the scope and spirit of the described embodiments. The various aspects and embodiments disclosed herein are for purposes of illustration and are not intended to be limiting, with the true scope and spirit being indicated by the following claims.

## Claims

1. A brake for a radiation therapy machine, the brake comprising:
braking arms;
an electric actuator, the electric actuator configured to cause the braking arms to engage a rotor of a radiation therapy machine based on a voltage applied to the electric actuator, the rotor being coupled to a gantry that is rotatable about an axis.

2. The brake of claim 1, wherein the electric actuator includes:
a plunger, and
a solenoid defining a channel to receive the plunger, the solenoid configured to cause the plunger to move based on a voltage applied to the electric actuator.

3. The brake of claim 2, including a linkage coupled to the plunger and the braking arms, the linkage configured to translate an axial force from the plunger to move the braking arms.

4. The brake of claim 2 or 3, wherein the electric actuator is configured to move a portion of the plunger into the channel when a first voltage is applied to the electric actuator and is configured to maintain a position of the portion plunger in the channel when a second voltage is applied to the electric actuator, the second voltage being less than the first voltage.

5. The brake of claim 1, 2, 3 or 4, wherein the electric actuator further includes,
a discharging circuit configured to discharge the voltage applied to the electric actuator, the discharging circuit including a Zener diode.

6. The brake of any one of claims 2 to 5, wherein the linkage is configured to translate a first axial force in a first direction into a second axial force in a second direction, the first axial force and the second axial force occurring at separate locations.

7. The brake of any one of claims 2 to 6, wherein the linkage includes a clevis, and:
(i) the at least one brake further includes a nut between the clevis and the plunger, wherein, optionally, the nut prevents motion of the clevis in a first direction relative to the plunger;
and/or
(ii) the at least one brake further includes a shaft, the clevis and the plunger coupled to the shaft such that a distance between the clevis and the plunger is adjustable.

8. The brake of any one of claims 1 to 7, including,
at least one spring between the braking arms, the at least one spring configured to provide a clamping force to the braking arms.

9. The brake of one of claims 1 to 8, including,
an override mechanism configured to cause the braking arms to disengage the rotor independent of the voltage applied to the electric actuator.

10. A radiation therapy machine comprising:
a gantry rotatable about an axis;
a rotor coupled to the gantry; and
at least one brake as claimed in one of claims 1 to 9.

11. The radiation therapy machine of claim 10, wherein the rotor is between the braking arms and/or wherein the electric actuator causes the braking arms to engage the rotor when the voltage applied to the electric actuator is zero volts.

12. The radiation therapy machine of claim 10 or 11, further comprising:
a stand; and
a drive system, the drive system coupling the stand to the gantry, the at least one brake being mounted to the drive system, wherein, optionally, the drive system includes a bearing assembly coupled to the gantry and the stand, the at least one brake being mounted to a portion of the bearing assembly.

13. The radiation therapy machine of claim 10, 11 or 12, wherein the at least one brake includes a plurality of brakes arranged around the rotor.

14. The radiation therapy machine of claim 10, 11, 12 or 13, further comprising:
processing circuitry configured to cause the radiation therapy machine to,
obtain a speed of the gantry,
estimate a stopping distance based on the speed of the gantry, and
determine whether to engage the at least one brake based on the estimated stopping distance, wherein, optionally, the estimated stopping distance corresponds to a motor stopping distance.

15. A non-transitory computer readable medium storing instructions, when executed by processing circuitry of a radiation therapy machine, cause the radiation therapy machine to,
obtain a braking signal;
estimate a stopping distance of a gantry;
determine if the estimated stopping distance is greater than a threshold; and
apply a brake if the estimated stopping distance is greater than the threshold.
